# EUROPEAN PATENT APPLICATION

(11) **EP 3 266 378 A1**
(43) Date of publication of application: **10.01.2018**
(21) Application number: 17180107.9
(22) Date of filing: 06.07.2017
(51) Int. Cl.: A61B 8/00, A61B 8/08, A61B 5/00

(54) **APPARATUS, METHOD, AND PROGRAM FOR OBTAINING INFORMATION DERIVED FROM ULTRASONIC WAVES AND PHOTOACOUSTIC WAVES**

(30) Priority: 08.07.2016 JP 2016136105
(71) Applicant: CANON KABUSHIKI KAISHA, Ohta-ku Tokyo 146-8501 (JP)
(72) Inventor: HAYASHI, Akinori, Ohta-ku, Tokyo 146-8501 (JP); MIYAZAWA, Nobu, Ohta-ku, Tokyo 146-8501 (JP); SEMBA, Daiya, Ohta-ku, Tokyo 146-8501 (JP); OKA, Kazuhito, Ohta-ku, Tokyo 146-8501 (JP)
(74) Representative: Houle, Timothy James

(57) **Abstract**

An apparatus according to an aspect of the present invention includes first obtaining means configured to obtain an ultrasound image generated by transmitting and receiving ultrasonic waves to and from an object, display control means configured to control display means to display the ultrasound image, second obtaining means configured to obtain a photoacoustic signal generated by receiving photoacoustic waves generated from light irradiated to the object, and saving control means configured to obtain information representing a save instruction given when the ultrasound image is being displayed, and configured to save in storage means the ultrasound image corresponding to a time point of the save instruction and information derived from the photoacoustic signal based on the information representing the save instruction.

## Description

### BACKGROUND OF THE INVENTION

### Field of the Invention

The present invention relates to an apparatus or a method for obtaining information derived from ultrasonic waves and photoacoustic waves.

### Description of the Related Art

An ultrasonic diagnostic apparatus which generates an ultrasound image by transmitting and receiving ultrasonic waves has been known as an image diagnostic apparatus which images an internal state of a living body noninvasively. An ultrasonic diagnostic apparatus generates an ultrasound image on the basis of a reception signal of transmitted waves or reflected waves (ultrasonic echo) of transmission ultrasonic waves.

Japanese Patent Laid-Open No. 2015-66318 discloses an ultrasonic diagnostic apparatus which generates an ultrasound image based on an ultrasonic echo. Japanese Patent Laid-Open No. 2015-66318 discloses that a freeze button, for example, may be operated to save an image displayed on a monitor.

On the other hand, a photoacoustic apparatus which applies an ultrasonic wave (photoacoustic wave) generated by biological tissue irradiated with light and adiabatically expanded due to optical energy of the irradiated light has been known as an image diagnostic apparatus which images an internal state of a living body noninvasively. Such a photoacoustic apparatus may generate a photoacoustic image based on a reception signal of photoacoustic waves.

Japanese Patent Laid-Open No. 2012-196430 discloses a switch for selecting an operation mode in which a reflected ultrasonic wave is detected or an operation mode in which photoacoustic waves are detected. Japanese Patent Laid-Open No. 2012-196430 discloses selecting display of an ultrasound image or display of a photoacoustic image by using the switch.

Japanese Patent Laid-Open No. 2012-196430 discloses a switch for selecting an ultrasound image, a photoacoustic image, or a superimposition image of an ultrasound image and a photoacoustic image and displaying the selected image on a display unit. However, a time point for saving those images is not disclosed.

### SUMMARY OF THE INVENTION

According to a saving method in an ultrasonic diagnostic apparatus in the past, a user may operate a freeze button, for example, when an image is displayed to save the image displayed on a monitor. In this case, after an ultrasound image is saved when the ultrasound image is being displayed, a user may need to save a photoacoustic image when the ultrasound image is being displayed. In such a case, the display image is to be changed to a photoacoustic image, and the photoacoustic image is to be saved when the photoacoustic image is being displayed.

In this case, a time lag for the change of the display image may occur during a period from a time when an ultrasound image is saved to a time when a photoacoustic image is saved, and there is a possibility that, during the time lag, an object thereof may move its body or a positional deviation of a probe therefor may occur. As a result, the photoacoustic image having a state different from a state found on the ultrasound image may be saved. In other words, a photoacoustic image having a state different from a state intended by a user is saved.

The present invention in its first aspect provides an apparatus as specified in claims 1 to 9.

The present invention in its second aspect provides a method as specified in claims 10 to 18.

The present invention in its third aspect provides a program as specified in claim 19.

Further features of the present invention will become apparent from the following description of exemplary embodiments with reference to the attached drawings. Each of the embodiments of the present invention described below can be implemented solely or as a combination of a plurality of the embodiments or features thereof where necessary or where the combination of elements or features from individual embodiments in a single embodiment is beneficial.

### BRIEF DESCRIPTION OF THE DRAWINGS

Fig. 1 is a block diagram illustrating an inspection system according to a first embodiment.
Fig. 2 is a schematic diagram illustrating a probe according to the first embodiment.
Fig. 3 is a configuration diagram illustrating a computer and peripherals therefor according to the first embodiment.
Fig. 4 is a flowchart illustrating a saving method according to the first embodiment.
Fig. 5 illustrates a data structure of saved data according to the first embodiment.
Fig. 6 is a timing chart according to the first embodiment.
Fig. 7 is another timing chart according to the first embodiment.
Fig. 8 is another timing chart according to the first embodiment.
Fig. 9 is another timing chart according to the first embodiment.
Fig. 10 is a flowchart illustrating a saving method according to a second embodiment.
Fig. 11 illustrates a timing chart according to the second embodiment.
Fig. 12 is another timing chart according to the second embodiment.
Fig. 13 is another timing chart according to the second embodiment.
Fig. 14 is another timing chart according to the second embodiment.
Fig. 15 illustrates a data structure of examination order information according to a third embodiment.

### DESCRIPTION OF THE EMBODIMENTS

For convenience of description, an acoustic wave generated by thermal expansion of an optical absorber irradiated with light will be called a photoacoustic wave, hereinafter. Furthermore, for convenience of description, an acoustic wave or a reflected wave (echo) transmitted from a transducer will be called an ultrasonic wave, hereinafter.

Use of a superimposition image of an ultrasound image and a photoacoustic image is considered as effective for diagnoses. Accordingly, it may also be considered as effective for diagnoses that obtaining and saving an ultrasound image and a photoacoustic image with a smaller time difference therebetween and displaying the images associated with each other in a superimposed or parallel arrangement.

On the other hand, like an ultrasonic diagnostic apparatus in the past, a user such as a doctor or a technician may prefer to instruct to save an ultrasound image by checking a display image thereof. In this case, if a photoacoustic image is superimposed on an ultrasound image, there is a possibility that the photoacoustic image may prevent a user from determining whether to instruct to save or not.

A saving method for an ultrasonic diagnostic apparatus in the past may require saving an ultrasound image first, then changing the display image from the ultrasound image to a photoacoustic image, and saving the photoacoustic image.

Accordingly, when an instruction to save (hereinafter, save instruction) is given when an ultrasound image is displayed when a photoacoustic image is not displayed, the present invention saves the photoacoustic image corresponding to the save instruction in addition to the ultrasound image corresponding to the save instruction. That is, the photoacoustic image and the ultrasound image are stored in a storage unit in response to the save instruction. For example, an ultrasound image displayed when a save instruction is given and a photoacoustic image neighboring in time to the time point when the ultrasound image is obtained are saved in association. Thus, when a user needs to save a photoacoustic image when checking an ultrasound image, the user can save both of the photoacoustic image and the ultrasound image without requiring changing the display image to the photoacoustic image. Therefore, the user can check a superimposition image of the photoacoustic image and the ultrasound image obtained with a small time difference therebetween even after an inspection.

### First Embodiment

Embodiments of the present invention will be described in detail below with reference to drawings. Like numbers refer to like parts throughout in principle, and any repetitive description will be omitted.

### Configuration of Inspection System

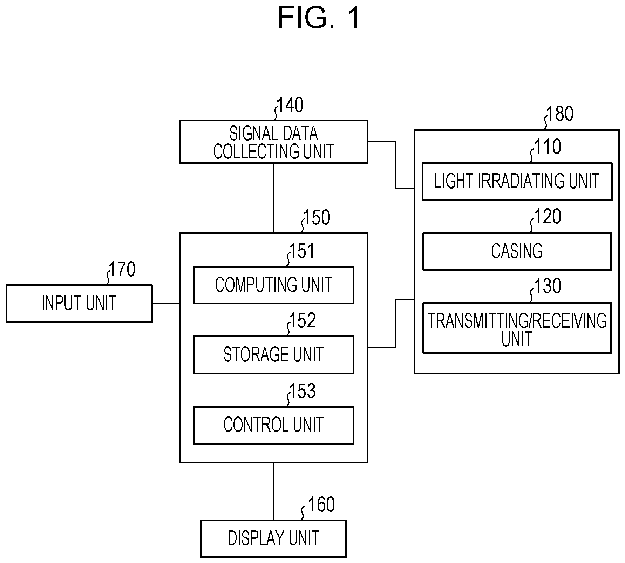

An inspection system according to a first embodiment will be schematically described with reference to Fig. 1. Fig. 1 is a schematic block diagram illustrating an overall inspection system. The inspection system according to this embodiment includes a signal data collecting unit 140, a computer 150, a display unit 160, an input unit 170, and a probe 180.

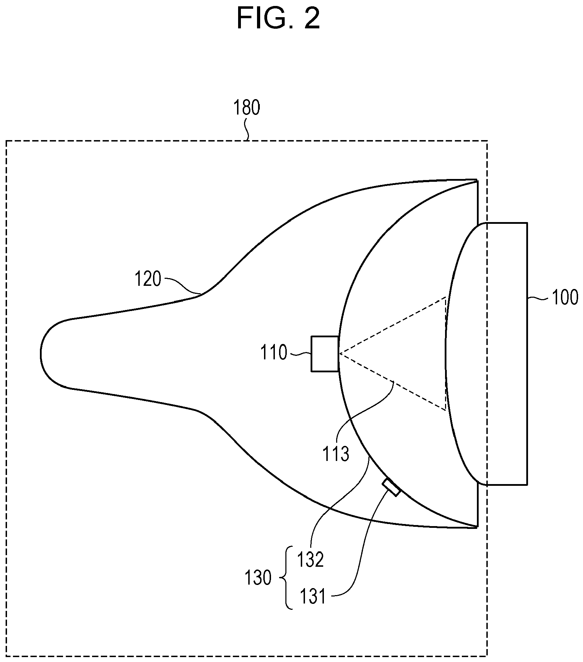

Fig. 2 is a schematic diagram of the probe 180 according to this embodiment. The probe 180 has a light irradiating unit 110, a casing 120 including a holding portion, and a transmitting/receiving unit 130. An object 100 is a measurement object.

The light irradiating unit 110 irradiates pulsed light 113 to the object 100 so that acoustic waves can occur within the object 100. An acoustic wave caused by light due to a photoacoustic effect will also be called a photoacoustic wave. The transmitting/receiving unit 130 is configured to receive photoacoustic waves and output an analog electric signal (photoacoustic signal). The transmitting/receiving unit 130 is further configured to transmit ultrasonic waves to the object 100 and receive echo waves of the transmitted ultrasonic waves to output an analog electric signal (ultrasonic signal).

The signal data collecting unit 140 is configured to convert an analog signal output from the transmitting/receiving unit 130 to a digital signal and output it to the computer 150. The computer 150 stores the digital signal output from the signal data collecting unit 140 as signal data derived from ultrasonic waves or photoacoustic waves.

The computer 150 is configured to perform signal processing on a stored digital signal to generate image data representing an ultrasound image or a photoacoustic image. The computer 150 performs an image process on the resulting image data and then outputs image data to the display unit 160. The display unit 160 is configured to display an ultrasound image or a photoacoustic image. A doctor or a technician as a user can perform diagnosis by checking an ultrasound image and a photoacoustic image displayed on the display unit 160. A display image is saved in a data management system connected to a memory within the computer 150 or to the inspection system over a network based on a save instruction from a user or the computer 150.

The computer 150 is configured to perform drive control over components included in the inspection system. The display unit 160 may display an image generated in the computer 150 and a GUI. The input unit 170 is configured to be usable by a user for inputting information. A user may use the input unit 170 to perform an operation such as instructing to save a display image.

A photoacoustic image obtained by the inspection system according to this embodiment is a concept including an image derived from photoacoustic waves generated from irradiated light. A photoacoustic image includes image data representing at least one spatial distribution of information regarding sound pressure for generating photoacoustic waves (initial sound pressure), an optical absorption energy density, an optical absorption coefficient, and a concentration of a substance contained in an object, for example. The information regarding a concentration of a substance may be an oxyhemoglobin concentration, a deoxyhemoglobin concentration, a total hemoglobin concentration, or an oxygen saturation, for example. The total hemoglobin concentration is a sum of an oxyhemoglobin concentration and a deoxyhemoglobin concentration. The oxygen saturation is a ratio of oxyhemoglobin to whole hemoglobin. The photoacoustic image is not limited to an image representing a spatial distribution but may be an image representing a numerical value. For example, the photoacoustic image is a concept including an image representing information derived from a photoacoustic signal, such as a photoacoustic signal (RAW data), an average concentration of a substance contained in an object, a pixel value at a specific position in a spatial distribution, or a statistic (such as an average value or a median value) of pixel values in a spatial distribution, for example. As a photoacoustic image, a numerical value of an average concentration of a substance contained in an object, for example, may be displayed on the display unit 160.

An ultrasound image obtained by the inspection system according to this embodiment includes image data of at least one of a B mode image, a doppler image, and an elastography image. The ultrasound image is a concept including an image obtained by transmitting and receiving ultrasonic waves.

Components of an object information obtaining apparatus according to this embodiment will be described in detail below.

### Light Irradiating Unit 110

The light irradiating unit 110 includes a light source configured to emit pulsed light 113, and an optical system configured to guide the pulsed light 113 emitted from the light source to the object 100. The pulsed light here includes so-called square-wave or triangle-wave light.

The light emitted from the light source may have a pulse width ranging from 1 ns to 100 ns. The light may have a wavelength ranging from 400 nm to 1600 nm. In order to image a blood vessel neighboring to a surface of a living body with a high resolution, light having a wavelength (ranging from 400 nm to 700 nm) which is largely absorbed by a blood vessel may be used. On the other hand, in order to image a deep part of a living body, light having a wavelength (ranging from 700 nm to 1100 nm) which is typically absorbed less by background tissue (such as water or fat) of a living body may be used.

The light source may be a laser or a light emitting diode, for example. Alternatively, the light source may be capable of performing wavelength conversion for measurement using light having a plurality of wavelengths. When light having a plurality of wavelengths is irradiated to an object, a plurality of light sources which emit light beams having wavelengths different from each other may be provided so that the light beams can be irradiated alternately from the light sources. A set of a plurality of light sources if used is also collectively called as a light source. Various lasers may be applied here such as a solid-state laser, a gas laser, a dye laser, and a semiconductor laser. For example, a pulsed laser such as an Nd:YAG laser and an alexandrite laser may be used as the light source 111. Alternatively, a Ti:sa laser or an OPO (Optical Parametric Oscillators) laser applying an Nd:YAG laser light as excited light may be used as the light source. A microwave source may be used as the light source instead.

The optical system may include optical elements such as a lens, a mirror, and optical fiber. In a case where a breast is the object 100, for example, pulsed light having an increased beam diameter is to be irradiated. Accordingly, the optical system may include a light emitting unit having a diffusing plate configured to diffuse light. On the other hand, a photoacoustic microscope may have an increased resolution with an optical system having a light emitting unit including a lens to irradiate a focused beam.

Alternatively, the pulsed light 113 may be irradiated from the light source directly to the object 100 by the light irradiating unit 110 without an optical system. The components of the light irradiating unit 110 such as the light source may be provided externally to the casing 120.

### Transmitting/Receiving Unit 130

The transmitting/receiving unit 130 includes a transducer 131 configured to output an electric signal from received acoustic waves, and a supporting member 132 configured to support the transducer 131. The transducer 131 is also capable of transmitting acoustic waves. Fig. 2 only illustrates one transducer 131 for simplicity, the transmitting/receiving unit 130 may include a plurality of transducers.

The transducer 131 may be formed of a piezoelectric ceramic material such as PZT (lead zirconate titanate) or a polymer piezoelectric film material such as PVDF (polyvinylidene difluoride), for example. An element excluding a piezoelectric element may be used instead. For example, capacitive micro-machined ultrasonic transducers, CMUT, or a transducer applying a Fabry-Perot interferometer may be used. Any kind of transducer may be adopted if it is capable of outputting an electric signal from received acoustic waves. A signal obtained by the transducer is a temporal resolution signal. In other words, a signal obtained by a receiving element has an amplitude representing a value (such as a value proportional to sound pressure) based on sound pressure received by the transducer at different times.

Photoacoustic waves contain frequency components typically ranging from 100 KHz to 100 MHz, and the transducer 131 is capable of detecting these frequencies.

The supporting member 132 may be formed of a metallic material having a high mechanical strength. For a case where a user holds the casing 120 to scan the probe 180, the supporting member 132 may be formed of a polymer material such as plastics from the view point of weight reduction. In order to launch more irradiation light into an object, the supporting member 132 may have a mirror surface or a surface processed to be light scattering closer to the object 100. According to this embodiment, the supporting member 132 has a hemispherical enclosure shape and is configured to support a plurality of transducers 131 on the hemispherical enclosure. In this case, the transducers 131 arranged on the supporting member 132 have directional axes gathering closely to the center of the curvature of the hemisphere. An image obtained by using a group of electric signals output from the plurality of transducers 131 has high image quality at a part produced by electric signals from the transducers around the center of curvature. The supporting member 132 may have any configuration if it can support the transducers 131. The supporting member 132 may have a plurality of transducers on its plane or curved surface such as a 1D array, a 1.5D array, a 1.75D array, and a 2D array.

The supporting member 132 may function as a container configured to reserve an acoustic matching material. In other words, the supporting member 132 may be a container for arranging an acoustic matching material between the transducer 131 and the object 100.

The transmitting/receiving unit 130 may include an amplifier configured to amplify time-series analog signals output from the transducers 131. The transmitting/receiving unit 130 may include an A/D converter configured to convert time-series analog signals output from the transducers 131 to time-series digital signals. In other words, the transmitting/receiving unit 130 may include a signal data collecting unit 140, which will be described below.

For detection of acoustic waves at various angles, the transducer 131 may be arranged to surround the entire perimeter of the object 100. However, in a case where it is difficult to arrange transducers to surround the entire perimeter of the object 100, the transducers may be arranged on the hemisphere supporting member to surround the entire perimeter as illustrated in Fig. 2.

The arrangement and number of transducers and the shape of the supporting member may be optimized in accordance with an object, and any kind of transmitting/receiving unit 130 may be adopted with respect to the present invention.

The space between the transmitting/receiving unit 130 and the object 100 is filled with a medium in which photoacoustic waves can propagate. The medium may be made of a material in which acoustic waves can propagate and which has an acoustic characteristic matching at an interface between the object 100 and the transducer 131 and has a transmittance of photoacoustic waves as high as possible. For example, the medium may be water or ultrasound gel.

It should be noted that a transducer configured to transmit ultrasonic waves and a transducer configured to receive acoustic waves may be provided separately. Alternatively, one transducer may be provided which is configured to transmit ultrasonic waves and receive acoustic waves. A transducer configured to transmit and receive ultrasonic waves and a transducer configured to receive photoacoustic waves may be provided separately. Alternatively, one transducer may be provided which is configured to transmit and receive ultrasonic waves and receive photoacoustic waves.

### Signal Data Collecting Unit 140

The signal data collecting unit 140 includes an amplifier configured to amplify an electric signal being an analog signal output from the transducer 131 and an A/D converter configured to convert an analog signal output from the amplifier to a digital signal. The signal data collecting unit 140 may be an FPGA (Field Programmable Gate Array) chip, for example. A digital signal output from the signal data collecting unit 140 is stored in a storage unit 152 within the computer 150. The signal data collecting unit 140 is also called a Data Acquisition System (DAS). The term "electric signal" herein refers to a concept including an analog signal and a digital signal. The signal data collecting unit 140 is connected to a light detection sensor attached to the light emitting unit in the light irradiating unit 110 and may start processing by being triggered by and synchronized with emission of the pulsed light 113 from the light irradiating unit 110. The signal data collecting unit 140 may start the processing by being triggered by and synchronized with a save instruction given by using a freeze button, which will be described below.

### Computer 150

The computer 150 includes a computing unit 151, the storage unit 152, and a control unit 153. These components have functions, which will be described with reference to a processing flow.

A unit responsible for a computing function as the computing unit 151 may have a processor such as a CPU and a GPU (Graphics Processing Unit) and a computing circuit such as an FPGA (Field Programmable Gate Array) chip. These units may include a plurality of processors and computing circuits, instead of a single processor and a single computing circuit. The computing unit 151 may process a reception signal in accordance with parameters such as the speed of sound of an object and a holding cup from the input unit 170.

The storage unit 152 may be a non-transitory storage medium such as a ROM (Read only memory), a magnetic disk and a flash memory. The storage unit 152 may be a volatile medium such as a RAM (Random Access Memory). A storage medium storing a program is a non-transitory storage medium.

The control unit 153 is configured by a computing element such as a CPU. The control unit 153 is configured to control operations performed by components of the photoacoustic apparatus. The control unit 153 may control the components of the inspection system in response to an instruction signal based on an operation such as a start of measurement given through the input unit 170. The control unit 153 may read out program code stored in the storage unit 152 and controls an operation performed by a component of the inspection system.

The computer 150 may be a specially designed workstation. The components of the computer 150 may be configured by different hardware modules. Alternatively, at least partial components of the computer 150 may be configured by a single hardware module.

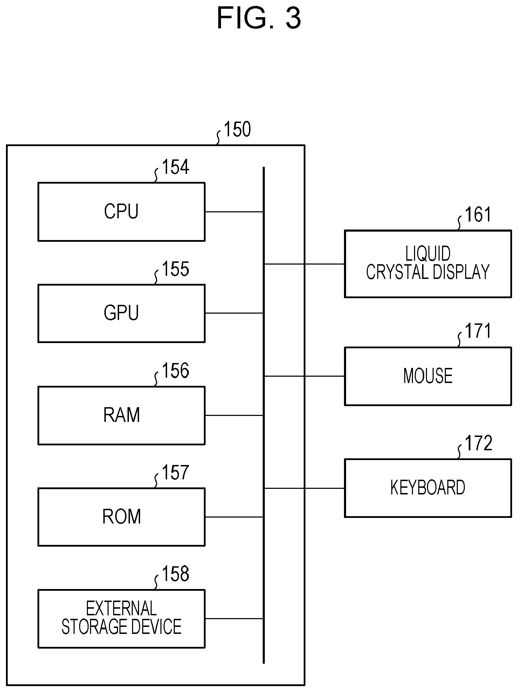

Fig. 3 illustrates a specific configuration example of the computer 150 according to this embodiment. The computer 150 according to this embodiment includes a CPU 154, a GPU 155, a RAM 156, a ROM 157, and an external storage device 158. A liquid crystal display 161 as the display unit 160 and a mouse 171 and a keyboard 172 as the input unit 170 are connected to the computer 150.

The computer 150 and the plurality of transducers 131 may be accommodated in a common casing. Alternatively, partial signal processing may be performed by the computer accommodated in the casing while the rest of the signal processing may be performed by a computer provided externally to the casing. In this case, the computers provided internally and externally to the casing may be collectively called a computer according to this embodiment.

### Display Unit 160

The display unit 160 is a display such as a liquid crystal display and an organic EL (Electro Luminescence). The display unit 160 is configured to display an image based on object information obtained by the computer 150 and a numerical value corresponding to a specific position therein. The display unit 160 may display a graphical user interface (GUI) usable for operating an image or the system. For display of object information, the display unit 160 or the computer 150 may perform an image process (such as adjustment of a luminance value) thereon.

### Input Unit 170

The input unit 170 may be an operating console which can be operated by a user and may include a mouse and a keyboard. The display unit 160 may include a touch panel so that the display unit 160 can also be used as the input unit 170. The input unit 170 may include a freeze button usable by a user for giving an instruction such as a save instruction, which will be described below.

The components of the inspection system may be provided as separate apparatuses or may be integrated to one system. Alternatively, at least partial components of the inspection system may be integrated to one apparatus.

### Object 100

The object 100 will be described below though it is not a component of the inspection system. The inspection system according to this embodiment is usable for purposes such as diagnoses of human or animal malignant tumors and blood vessel diseases and follow-ups of chemical treatments. Therefore, the object 100 is assumed as a region to be diagnosed such as a living body, more specifically, the limbs including the breast, the neck, the abdomen, a finger and a toe of a human body or an animal. For example, in a case where a human body is a measurement object, oxyhemoglobin or deoxyhemoglobin or a blood vessel mostly including them or a neovessel formed in neighborhood of a tumor may be an optical absorber. Plaque of a carotid artery wall may be an optical absorber. Alternatively, a pigment such as methylene blue (MB), indocyanine green (ICG), gold minute particles, or an externally introduced substance integrating or chemically modifying them may be an optical absorber.

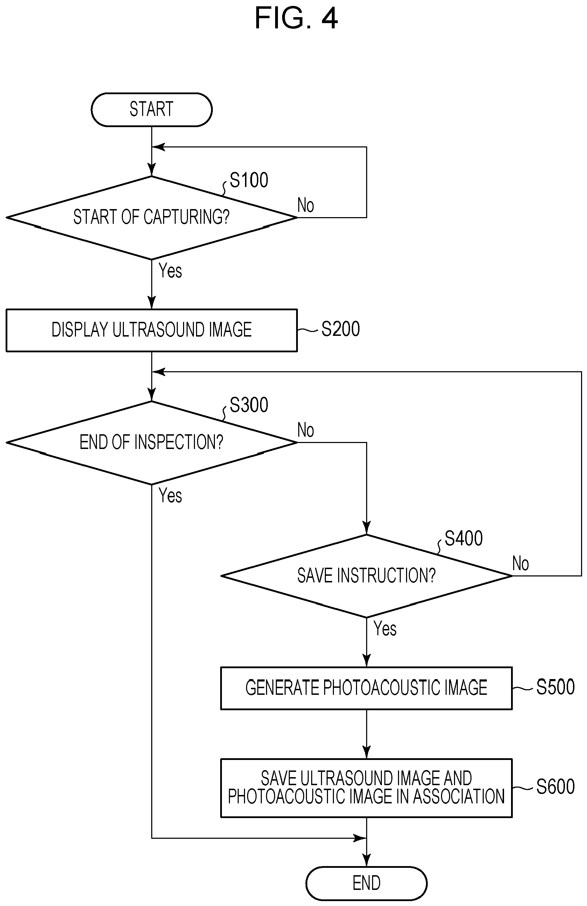

Next, with reference to Fig. 4, a control method for saving a photoacoustic image and an ultrasound image according to this embodiment will be described.

### S100: Determining Whether Start Of Capturing Is Instructed Or Not

The control unit 153 can receive an instruction to start capturing an ultrasound image. If the control unit 153 receives an instruction to start capturing, the processing moves to S200.

When a user instructs to start capturing an ultrasound image by using the input unit 170, the control unit 153 receives information representing the instruction to start capturing (hereinafter, capturing start instruction) from the input unit 170. For example, when a user presses a switch for capturing start provided in the probe 180, the control unit 153 receives information representing the capturing start instruction from the input unit 170.

In this processing, not only an instruction to capture an ultrasound image but also an instruction to capture both of an ultrasound image and a photoacoustic image may be received.

### S200: Displaying Ultrasound Image

The control unit 153 in response to the information representing capturing start performs the following device control.

The probe 180 transmits and receives ultrasonic waves to and from the object 100 to output an ultrasonic signal. The signal data collecting unit 140 performs analog-digital (AD) conversion processing on the ultrasonic signal and transmits the processed ultrasonic signal to the computer 150. The ultrasonic signal being a digital signal is stored in the storage unit 152. The computing unit 151 may perform reconstruction processing including phasing addition (Delay and Sum) on the ultrasonic signal to generate an ultrasound image. When the ultrasound image is generated, the ultrasonic signal saved in the storage unit 152 may be deleted. The control unit 153 being display control means transmits the generated ultrasound image to the display unit 160 and performs display control to control the display unit 160 to display the ultrasound image. This processing is repeatedly performed so that the ultrasound image to be displayed by the display unit 160 may be updated. Thus, the ultrasound image can be displayed as a moving image.

In this case, saving all of the ultrasound images displayed as a moving image by the display unit 160 in the storage unit 152 may greatly increase the saved data amount. In order to avoid the problem, when the display image is updated, the previously displayed ultrasound image may be deleted from the storage unit 152. However, in a case where an ultrasound image corresponding to a save instruction, which will be described below, is based on an ultrasound image generated before the save instruction, the ultrasound image may be saved because it is possibly to be saved.

In this processing, a photoacoustic image is not displayed over an ultrasound image. A photoacoustic image may be displayed on the display unit 160 if an ultrasound image can be separately observed. For example, an ultrasound image and a photoacoustic image may be displayed side by side so that the ultrasound image can be separately observed. However, in a case where the display region of the display unit 160 becomes small because of display of a photoacoustic image, an ultrasound image may only be displayed without display of a photoacoustic image.

In addition to a display mode in which a photoacoustic image is not superimposed on an ultrasound image as in the aforementioned processing, another display mode may be provided in which an ultrasound image and a photoacoustic image are superimposed to display them as a moving image. In this case, the control unit 153 may be configured to switch the display mode in response to a switching instruction given by a user through the input unit 170. For example, the control unit 153 may be configured to switch between the parallel display mode as a display mode preventing a photoacoustic image from being superimposed on an ultrasound image and the superimposition mode.

### S300: Determining Whether End Of Inspection Is Instructed Or Not

The control unit 153 can receive an instruction to complete an inspection (hereinafter, inspection end instruction). The control unit 153 completes the inspection in response to an inspection end instruction. The control unit 153 can receive the instruction from a user or from an external network such as a hospital information system (HIS) and a radiology information system. The control unit 153 may determine end of an inspection at a time after a lapse of a predetermined time period from the inspection start instruction received in S100.

### S400: Determining Whether Save Instruction Is Given Or Not

The control unit 153 can receive a save instruction. When the control unit 153 receives a save instruction, the processing moves to S500.

A user may observe ultrasound images displayed as a moving image on the display unit 160 and can give a save instruction by using the input unit 170 when an object to be saved is found among the ultrasound images. In this case, when the display unit 160 displays a still image, a user may instruct to save the image by pressing a freeze button provided in an operating console as the input unit 170, for example. Here, the control unit 153 receives information representing a save instruction from the input unit 170.

The computing unit 151 may perform an image process on the ultrasound image generated in S200 to generate information representing a save instruction if the ultrasound image includes a region of interest and may transmit the information to the control unit 153. For example, when a region of interest is determined based on a user's instruction or an examination order, the computing unit 151 reads out a prestored image pattern corresponding to the region of interest from the storage unit 152 and correlates the image pattern and the ultrasound image generated in S100. The computing unit 151 determines the ultrasound image to be saved if the calculated correlation is higher than a threshold value and generates information representing a save instruction.

The control unit 153 may receive a save instruction from an external network such as an HIS and an RIS.

### S500: Generating Photoacoustic image

If the control unit 153 receives information representing a save instruction, the control unit 153 may perform the following device controls.

First of all, if the control unit 153 receives information representing a save instruction, the control unit 153 transmits information (control signal) representing light irradiation to the probe 180. The probe 180 having received the information representing light irradiation irradiates light to the object 100, receives photoacoustic waves caused by the light irradiation and outputs a photoacoustic signal. The signal data collecting unit 140 may perform AD conversion processing on the photoacoustic signal and transmit the processed photoacoustic signal to the computer 150. The photoacoustic signal being a digital signal is stored in the storage unit 152. The computing unit 151 may perform reconstruction processing such as Universal Back-Projection (UBP) on the photoacoustic signal to generate a photoacoustic image. Here, a reconstruction region of the photoacoustic image may be an ultrasound image display region displayed when a save instruction is given. In other words, the computing unit 151 may receive information regarding an ultrasound image display region displayed when a save instruction is given and determine a reconstruction region based on the information. When the photoacoustic image is generated, the photoacoustic signal saved in the storage unit 152 may be deleted. However, this is not applicable if the photoacoustic signal is to be used in a process, which will be described below. The inspection system according to this embodiment can be triggered to perform light irradiation by information representing a save instruction to generate a photoacoustic image corresponding to the time point of the save instruction. The probe 180 may perform the light irradiation at a time point of a save instruction or after a lapse of a predetermined time point from a save instruction.

The control unit 153 may control the components to perform light irradiation during a period when it can be determined that there is less influence of a body movement due to breathing or pulsation, instead of in response to a save instruction, to generate a photoacoustic image. For example, the control unit 153 may control the light irradiating unit 110 to perform light irradiation within 250 ms from a save instruction. The control unit 153 may control the light irradiating unit 110 to perform light irradiation within 100 ms from a save instruction. The time period from a save instruction to light irradiation may be equal to a predetermined value or may be designated by a user by using the input unit 170. The control unit 153 may control the time point for light irradiation such that t1 < t2 and |t1 - t2| ≤ α are satisfied where t1 is a clock time of an image save instruction, t2 is a clock time of light irradiation for obtaining a photoacoustic signal, and α is a predetermined value. Alternatively, the control unit 153 may control a time point for light irradiation such that t1 > t2 and |t1 - t2| ≤ α can be satisfied. The predetermined value α may be designated by a user by using the input unit 170.

The control unit 153 may control to perform light irradiation when receiving information describing that it is detected that the probe 180 and the object 100 are brought into contact in addition to information representing a save instruction. This can prevent light irradiation from occurring when the probe 180 and the object 100 are not in contact so that redundant light irradiation can be inhibited.

### S600: Saving Ultrasound Image and Photoacoustic image in Association

When the control unit 153 as saving control means receives information representing a save instruction, the control unit 153 saves an ultrasound image corresponding to the time point of the save instruction and a photoacoustic image generated by being triggered by the save instruction in S500. The photoacoustic image generated by being triggered by a save instruction in S500 corresponds to the photoacoustic image corresponding to the time point of the save instruction. An ultrasound image corresponding to the time point of a save instruction will be described below.

The storage unit 152 may save the ultrasound image displayed on the display unit 160 when a save instruction is received as an ultrasound image corresponding to the time point of the save instruction. The storage unit 152 may save an ultrasound image in a frame neighboring in time to the ultrasound image displayed on the display unit 160 when a save instruction is received as an ultrasound image corresponding to the time point of the save instruction.

An ultrasound image generated during a period when it can be determined that there is less influence of a body movement due to breathing or pulsation, instead of in response to a save instruction, may be saved as an ultrasound image in a frame neighboring in time. For example, the storage unit 152 may save an ultrasound image in a frame less than or equal to ±250 ms from a save instruction as an ultrasound image in a frame neighboring in time. The storage unit 152 may save an ultrasound image in a frame less than or equal to ±100 ms from a save instruction as an ultrasound image in a frame neighboring in time. The target to be saved may be determined with reference to the number of frames. For example, the storage unit 152 may save an ultrasound image less than or equal to ±5 frames from a save instruction as an ultrasound image in a frame neighboring in time. The storage unit 152 may save an ultrasound image within ±1 frame from a save instruction, that is, an adjacent ultrasound image as an ultrasound image in a frame neighboring in time. A time difference or a frame difference between a time point of a save instruction as described above and a time point for obtaining an image to be saved may be equal to a predetermined value or may be designated by a user by using the input unit 170. In other words, a user may use the input unit 170 to designate a range of "neighboring in time".

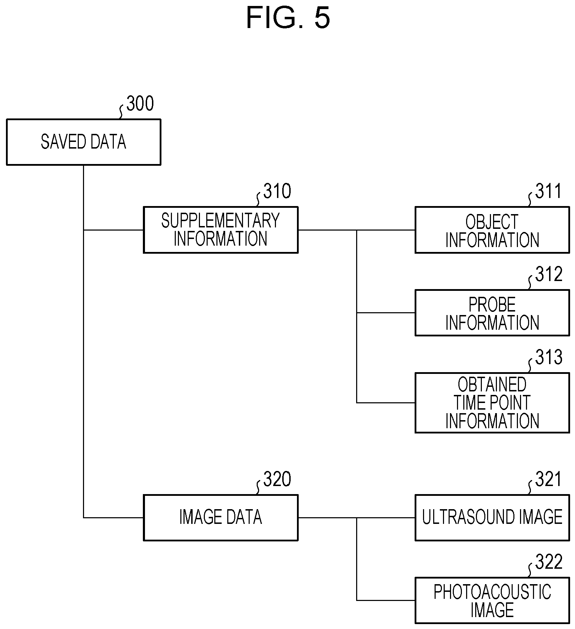

Having described that this processing saves an ultrasound image and a photoacoustic image in association, supplementary information may additionally be saved in association with them. For example, in S600, saved data 300 as illustrated in Fig. 5 can be stored in the storage unit 152. The saved data 300 may include supplementary information 310 and image data 320. The image data 320 may include an ultrasound image 321 and a photoacoustic image 322 that are in association with each other. The supplementary information 310 may include object information 311 being information regarding an object 100 and probe information 312 being information regarding the probe 180. The supplementary information 310 includes acquisition time point information 313 being information regarding an acquisition time point (acquisition clock time) of the ultrasound image 321 or the photoacoustic image 322 to be saved in S600.

The object information 311 may include at least one information piece of, for example, object ID, object name, age, blood pressure, heart rate, body temperature, height, weight, medical history, the number of weeks of pregnancy, and an inspection objective region. The inspection system may have an electrocardiographic apparatus or a pulse oximeter (not illustrated) and save information output from the electrocardiographic apparatus or the pulse oximeter at the time point of a save instruction in association therewith as object information. Furthermore, all information regarding an object may be saved as object information.

The probe information 312 includes information regarding the probe 180 such as a position and a gradient of the probe 180. For example, the probe 180 may have a position sensor such as a magnetic sensor, and information regarding an output from the position sensor corresponding to a time point of a save instruction may be saved as the probe information 312.

Information regarding a transmission time point for a control signal for transmission or reception of ultrasonic waves may be saved as the ultrasound image acquisition time point information 313. Information regarding a transmission time point for a control signal for light irradiation may be saved as photoacoustic image acquisition time point information. The inspection system may have a light detecting unit configured to detect pulsed light 113 ejected from the light irradiating unit 110 so that information regarding an output time point of a signal from the light detecting unit can be saved as photoacoustic image acquisition time point information.

Having described with reference to Fig. 5 the saved data 300 including a pair of image data pieces 320 that are associated with each other, a plurality of pairs of image data pieces may be included in one saved data set. In this case, supplementary information regarding a plurality of pairs of image data may also be saved in one saved data set. Alternatively, a plurality of pairs of image data pieces may be saved as different saved data sets. A plurality of image data pieces to be associated may be stored in one data file to associate the plurality of image data pieces. Supplementary information representing which images are to be associated may be attached to image data pieces so that a plurality of image data pieces can be associated.

The saved data may have a data format based on DICOM standard, for example. The format of saved data according to the present invention is not limited to DICOM but may be any data format.

According to this embodiment, in response to a save instruction given when a photoacoustic image is not displayed, an ultrasound image corresponding to the save instruction and a photoacoustic image corresponding to the save instruction may be saved in association. Thus, without switching from ultrasound image display to photoacoustic image display, the photoacoustic image can be saved. This can reduce the time lag from confirmation of a region of interest in the ultrasound image to saving the photoacoustic image. Because, according to this embodiment, light irradiation is triggered by a save instruction, redundant light irradiation can be inhibited. This can further suppress power consumption due to redundant light irradiation.

According to this embodiment, a photoacoustic image is saved in association with an ultrasound image. However, without limiting to a photoacoustic image as information representing a spatial distribution, information derived from a photoacoustic signal can be saved in association therewith. For example, a photoacoustic signal (RAW data) itself, an average concentration of a substance contained in an object, a pixel value at a specific position in a spatial distribution, or a statistic value (such as an average value or a median value) of pixel values in the spatial distribution may be associated with an ultrasound image as information derived from a photoacoustic signal.

After an ultrasound image and a photoacoustic image are associated based on a save instruction, the associated images may be superimposed for display on the display unit 160. The display of the resulting superimposition image may be triggered by a save instruction or may be executed based on an instruction from a user.

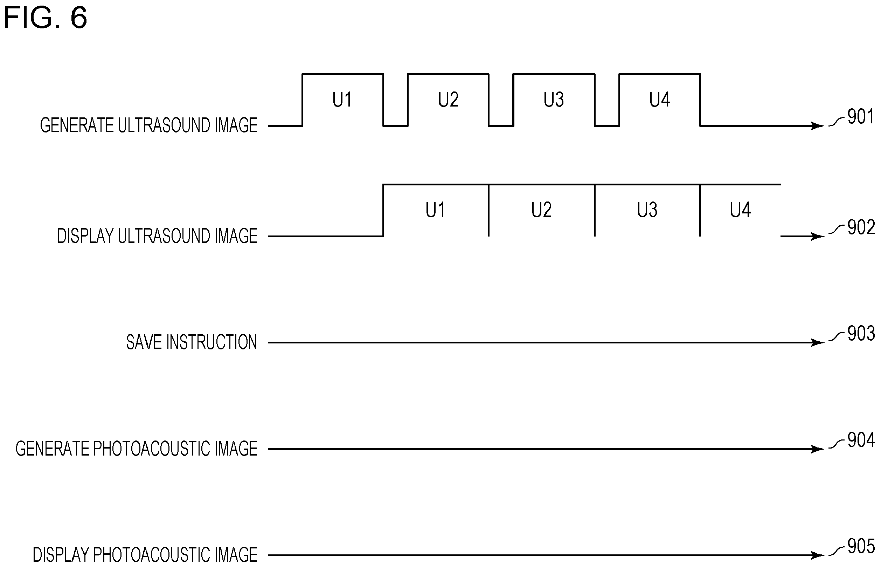

Next, with reference to Figs. 6 to 8, a measurement sequence according to this embodiment will be described. Each of diagrams 901 to 905 has a time axis horizontally where time passes as it goes to the right.

The diagram 901 illustrates timing for generating an ultrasound image. Transmission of ultrasonic waves starts at rises in the diagram 901, and generation of an ultrasound image completes at drops in the diagram 901. The diagram 902 illustrates ultrasound image display timing. When generation of an ultrasound image completes, display of the ultrasound image is enabled. The processing in S200 corresponds to the diagrams 901 and 902.

The diagram 903 illustrates timing of a save instruction. A rise in the diagram 903 indicates a time point when a save instruction is received. The processing in S400 corresponds to the diagram 903.

The diagram 904 illustrates timing for generating a photoacoustic image. Light irradiation starts at a rise in the diagram 904, and generation of a photoacoustic image completes at a drop in the diagram 904. The processing in S500 corresponds to the diagram 904.

The diagram 905 illustrates timing for displaying a photoacoustic image. When generation of a photoacoustic image completes, display of the photoacoustic image is enabled.

Fig. 6 is a timing chart where no save instruction is given. When no save instruction is given, ultrasonic waves are transmitted and are received, and, when generation of an ultrasound image completes, processing of updating the displayed ultrasound image is repeated. In other words, ultrasound images U1, U2, U3, and U4 are displayed in order of ultrasound images U1, U2, U3, and U4 as a moving image. In this case, neither light irradiation nor photoacoustic image generation is performed.

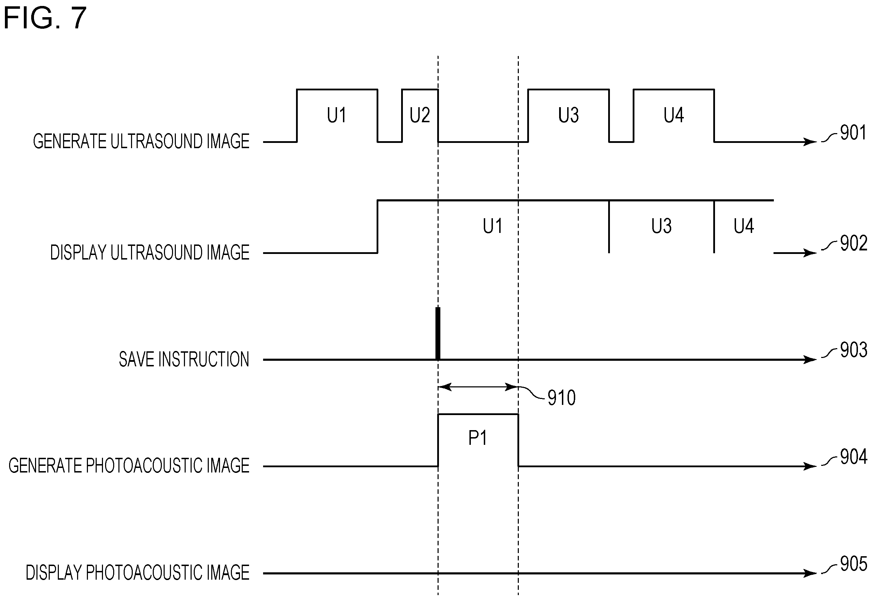

Fig. 7 is a timing chart where a save instruction is received when the ultrasound image U1 is being displayed. In this case, generation of the ultrasound image U2 is discontinued after a save instruction is received, and generation of a photoacoustic image P1 starts. After the generation of the photoacoustic image P1 completes, the ultrasound image U1 and the photoacoustic image P1 are saved in association. Thus, the photoacoustic image P1 can be generated without a long time from generation of the ultrasound image U1 to be saved, and the ultrasound image U1 and the photoacoustic image P1 can be saved in association.

As described above, an ultrasound image corresponding to the time point of a save instruction may be saved in association with an ultrasound image other than the ultrasound image U1. This is also true in the following case.

Light may be irradiated a plurality of number of times during a period 910 for generation of a photoacoustic image P1 having a high S/N, and the photoacoustic signals corresponding to the plurality of number of times of light irradiation may be used to generate the photoacoustic image P1. This is also true in the following case. Because the generation of the ultrasound image U2 is discontinued halfway, the display of the ultrasound image U1 may be continued when the photoacoustic image P1 is being generated and when the ultrasound image U3 is being generated.

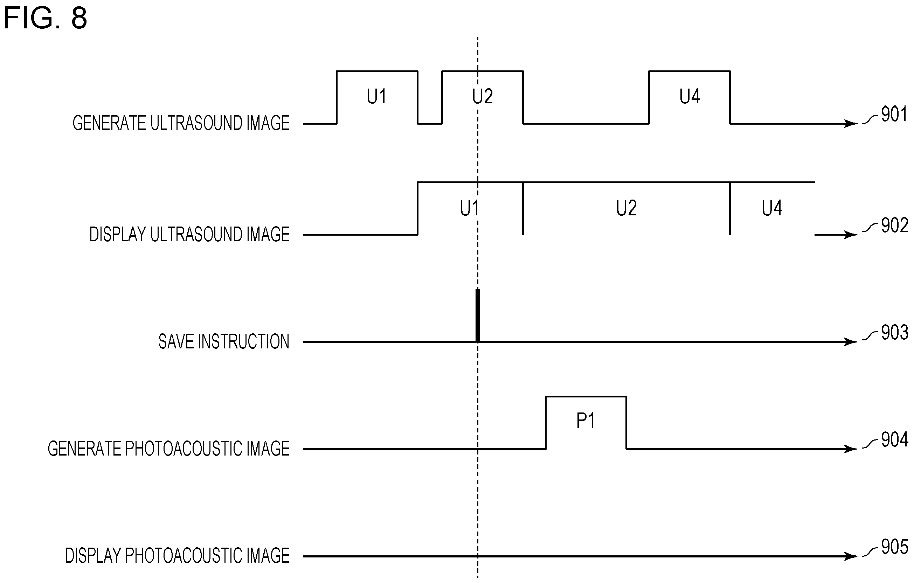

Fig. 8 is another timing chart in a case where a save instruction is received when the ultrasound image U1 is being displayed. Unlike Fig. 7, the generation of the ultrasound image U2 is continued when the save instruction is received, instead of discontinuing the generation of the ultrasound image U2. After the generation of the ultrasound image U2 completes, the generation of the photoacoustic image P1 starts. Also in this case, the photoacoustic image P1 and the ultrasound image U1 can be saved in association. The ultrasound image U2 being an ultrasound image corresponding to the time point of the save instruction and the photoacoustic image P1 may be saved in association. In this case, images that are closer in time can be saved in association, compared with saving in association with the ultrasound image U1.

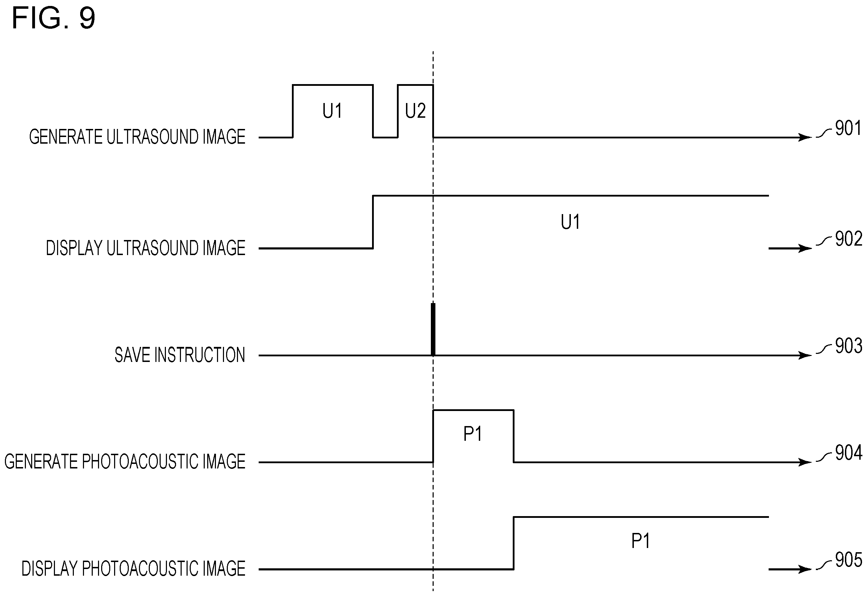

Fig. 9 is another timing chart in a case where a save instruction is received when the ultrasound image U1 is being displayed. In this case, when the save instruction is received, the ultrasound image U1 and photoacoustic image P1 are superimposed to display a still image. More specifically, when the save instruction is received, the display of the ultrasound image P1 is continued and, at the same time, the save instruction triggers to start the generation of the photoacoustic image P1. When the generation of the photoacoustic image P1 completes, the ultrasound image U1 and the photoacoustic image P1 are saved in association with each other, and the photoacoustic image P1 in association with the ultrasound image U1 being displayed is superimposed thereon for display. Thus, a user can perform diagnosis by watching the still image having the ultrasound image U1 and photoacoustic image P1 that are saved in association.

In a case where a save instruction is given in a display mode in which an ultrasound image and a photoacoustic image are superimposed for display as a moving image as described with reference to S300, the ultrasound image and photoacoustic image displayed on the display unit 160 when the save instruction is given may be saved in association with each other.

### Second Embodiment

According to the first embodiment, a save instruction triggers to start light irradiation and generation of a photoacoustic image, and the resulting photoacoustic image is saved in association with an ultrasound image. On the other hand, according to a second embodiment, a photoacoustic image which is obtained based on a photoacoustic image generated at a predetermined time point and which corresponds to the time point of the save instruction is saved in association with an ultrasound image.

This embodiment will also be described with reference to the inspection system according to the first embodiment. Like numbers refer to like parts in principle, and any repetitive description will be omitted.

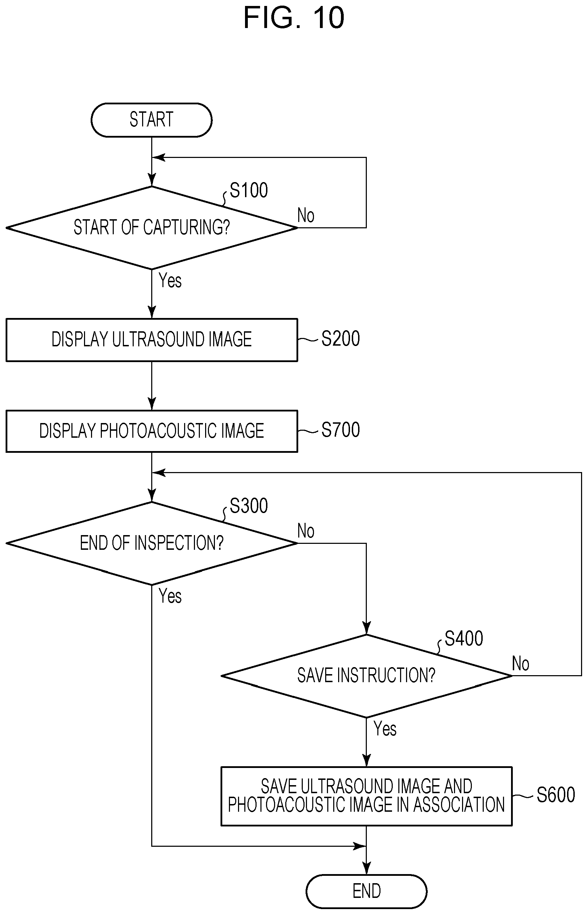

With reference to a flowchart illustrated in Fig. 10, a method for saving an ultrasound image and a photoacoustic image according to the second embodiment will be described. Like numbers refer to like steps, and any repetitive description will be omitted.

### S700: Generating Photoacoustic image

An inspection system according to this embodiment, light irradiation is performed at a predetermined time point, and a photoacoustic signal is obtained so that the photoacoustic signal can be used to generate a photoacoustic image. For example, the inspection system performs light irradiation in accordance with a repetition frequency of a light source and generates a photoacoustic image at the repetition frequency. The inspection system may further generate one photoacoustic image by performing light irradiation a plurality of number of times.

In order to prevent increases of the saved data amount, the storage unit 152 may save one photoacoustic image only. In other words, every time a new photoacoustic image is generated, the photoacoustic image to be saved in the storage unit 152 is updated therewith. The lastly saved photoacoustic image may be deleted from the storage unit 152. However, in a case where a photoacoustic image corresponding to a time point of a save instruction, which will be described below, is based on a photoacoustic image generated before the time point of the save instruction, the photoacoustic image may be saved because it is possibly to be saved. When the ultrasound image to be displayed on the display unit 160 is updated, the photoacoustic image to be saved in the storage unit 152 may be updated.

When a photoacoustic image is generated, the photoacoustic signal saved in the storage unit 152 may be deleted. However, the deletion may be performed except in cases where the photoacoustic signal is to be used in a process, which will be described below.

The processing in S700 may be performed before the processing in S200. Also in this case, the superimposition of a photoacoustic image on an ultrasound image is not performed in S200.

This processing may obtain information derived from a photoacoustic signal, without limiting to a photoacoustic image functioning as information representing a spatial distribution of object information. In other words, this processing may generate a photoacoustic image functioning as information representing a spatial distribution of object information. For example, a photoacoustic signal (RAW data) itself, an average concentration of a substance contained in an object, a pixel value at a specific position in a spatial distribution, or a statistic value (such as an average value or a median value) of pixel values in the spatial distribution may be obtained as information derived from a photoacoustic signal. The time point for obtaining a photoacoustic image corresponds to the light irradiation time point for obtaining the photoacoustic signal. It is assumed hereinafter that saving a photoacoustic image includes saving information derived from a photoacoustic signal.

### S900: Saving Ultrasound Image and Photoacoustic image in Association

When the control unit 153 according to this embodiment receives information representing a save instruction, the control unit 153 saves an ultrasound image and a photoacoustic image corresponding to the time point of the save instruction in association. The same processing as that of the first embodiment is performed on an ultrasound image corresponding to the time point of a save instruction. The photoacoustic image corresponding to the time point of the save instruction will be described below.

According to this embodiment, the control unit 153 obtains a photoacoustic image corresponding to the time point of a save instruction based on a photoacoustic image neighboring in time to the time point of the save instruction among photoacoustic images generated in S700. For example, the control unit 153 may use a photoacoustic image generated during a period when it can be determined that there is less influence of a body movement due to breathing or pulsation in response to a save instruction as a photoacoustic image in a frame neighboring in time. For example, the storage unit 152 may save a photoacoustic image in a frame within ±250 ms from a save instruction as a photoacoustic image in a frame neighboring in time. The storage unit 152 may save a photoacoustic image in a frame within ±100 ms from a save instruction as a photoacoustic image in a frame neighboring in time. A photoacoustic image to be saved may be determined with reference to the number of frames. For example, the storage unit 152 may save a photoacoustic image within ±5 frames from a save instruction as a photoacoustic image in a frame neighboring in time. The storage unit 152 may save a photoacoustic image within ±1 frame from or a photoacoustic image adjacent to a save instruction as a photoacoustic image in a frame neighboring in time. A time difference or a frame difference between a time point of a save instruction as described above and a time point for obtaining an image to be saved may be a predetermined value or may be designated by a user by using the input unit 170. In other words, a user may use the input unit 170 to designate a range of "neighboring in time". The control unit 153 may determine a photoacoustic image to be saved such that t1 < t2 and |t1 - t2| ≤ α are satisfied where t1 is a clock time of an image save instruction, t2 is a clock time of a time point for obtaining an photoacoustic image to be saved, and α is a predetermined value. Alternatively, the control unit 153 may determine a photoacoustic image to be saved such that t1 > t2 and |t1 - t2| ≤ α are satisfied. The predetermined value α may be designated by a user by using the input unit 170.

Photoacoustic images in a plurality of frames neighboring in time may be synthesized to obtain a photoacoustic image to be saved in association. The control unit 153 can obtain a photoacoustic image to be saved by synthesizing photoacoustic images in a plurality of frames by simple addition, addition average, weighting addition, or weighting addition average, for example. Some types of the synthesizing processing may be performed by the computing unit 151 as in other types of processing.

This processing may obtain not only a photoacoustic image to be saved but also information derived from a photoacoustic signal corresponding to the time point of a save instruction. The ultrasound image corresponding to the time point of the save instruction and the information derived from the photoacoustic signal corresponding to the time point of the save instruction may then be saved in association with each other. The computing unit 151 may synthesize information pieces derived from photoacoustic signals corresponding to a plurality of number of times of light irradiation to generate synthesized information, like the synthesizing processing.

The present invention may not save a photoacoustic image and an ultrasound image that are associated with each other in a storage unit in the inspection system. The control unit may save a photoacoustic image and an ultrasound image that are associated with each other in an image management system such as a PACS (Picture Archiving and Communication System) connected to an external network.

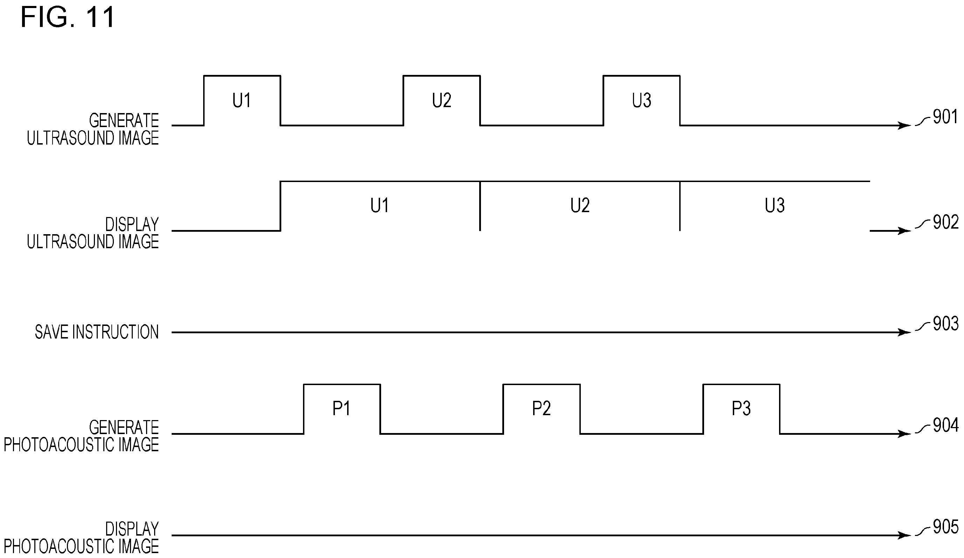

Next, with reference to Figs. 11 to 14, a measurement sequence according to this embodiment will be described. A diagram 901 illustrates timing for generating an ultrasound image. A diagram 902 illustrates ultrasound image display timing. When generation of an ultrasound image completes, display of the ultrasound image is enabled. A diagram 903 illustrates timing of a save instruction. A diagram 904 illustrates timing for generating a photoacoustic image. The processing in S700 corresponds to the diagram 904. A diagram 905 illustrates timing for displaying a photoacoustic image. When generation of a photoacoustic image completes, display of the photoacoustic image is enabled.

Fig. 11 is a timing chart where no save instruction is given. When no save instruction is given, ultrasonic waves are transmitted and are received, and, when generation of an ultrasound image completes, processing of updating the displayed ultrasound image is repeated. In other words, ultrasound images U1, U2, U3, and U4 are displayed in order of ultrasound images U1, U2, U3, and U4 as a moving image. On the other hand, a photoacoustic image is generated between generations of an ultrasound image. In other words, generation of an ultrasound image and generation of a photoacoustic image are executed alternately. In this case, a photoacoustic image is generated, but the photoacoustic image is not saved and displayed.

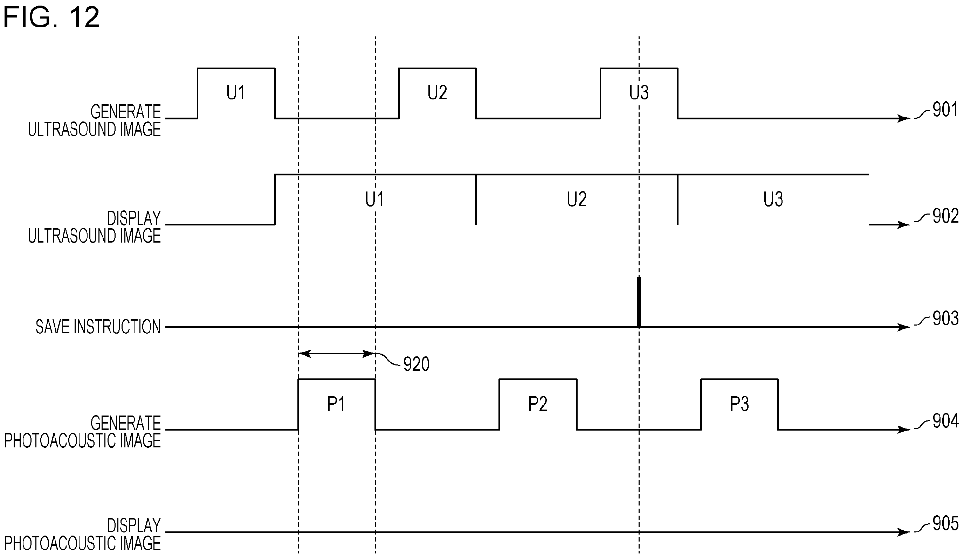

Fig. 12 is a timing chart in a case where a save instruction is received when the ultrasound image U2 is being displayed. In this case, the ultrasound image U2 displayed when a save instruction is received and the photoacoustic image P1 or the photoacoustic image P2 adjacent in time to the ultrasound image U2 can be saved in association. A photoacoustic image corresponding to light irradiation closer in time to transmission and reception of ultrasonic waves for generation of the ultrasound image U2 may be saved in association with the ultrasound image U2. Alternatively, a composition image of the photoacoustic image P1 and the photoacoustic image P2 and the ultrasound image U1 may be saved in association.

The ultrasound image U1 or the ultrasound image U3 neighboring in time to the ultrasound image U2 may be saved. In this case, a photoacoustic image neighboring in time to the ultrasound image U1 or the ultrasound image U3 may be saved.

Having described that the photoacoustic image P1 is generated during the period 920, a photoacoustic signal may only be obtained during the period 920 without generation of the photoacoustic image P1. In this case, the computing unit 151 may use a photoacoustic signal obtained during the period 920 after a save instruction is received to generate the photoacoustic image P1 and save the photoacoustic image P1 in association with the ultrasound image U2. During the period 920, instead of the photoacoustic image P1, information derived from a photoacoustic signal may be generated and be saved in association with the ultrasound image U2. These are also true for other photoacoustic images.

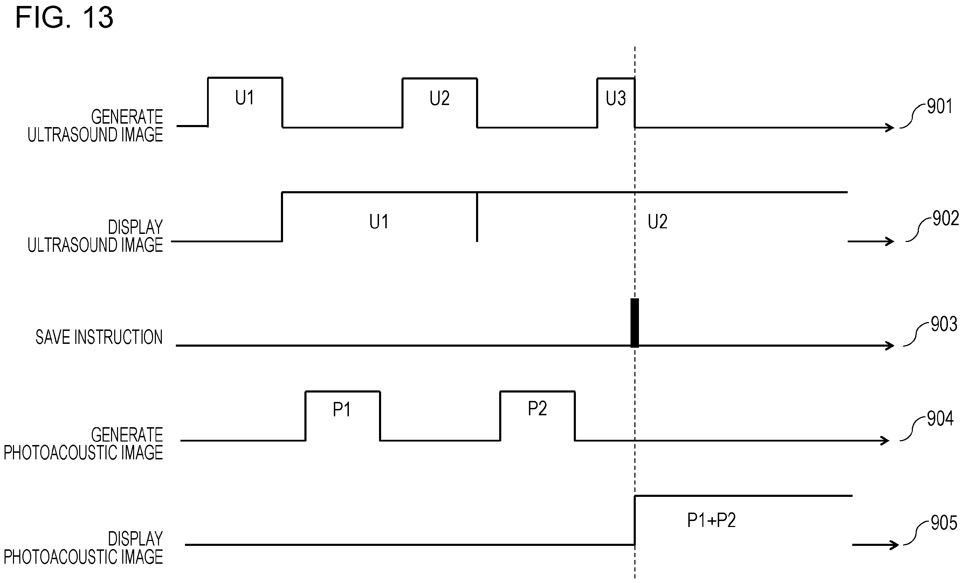

Fig. 13 is another timing chart in a case where a save instruction is received when the ultrasound image U2 is being displayed. In this case, when a save instruction is received, the ultrasound image U2 and a photoacoustic image P1+P2, being a composition image of the photoacoustic image P1 and the photoacoustic image P2, is saved.

Furthermore, in this case, a still image of the ultrasound image U2 displayed upon reception of a save instruction is continuously displayed. The generation of the ultrasound image U3 is discontinued. In other words, a save instruction triggers to switch from moving image display to still image display. Furthermore, in this case, a still image of the photoacoustic image P1+P2 saved in association with the ultrasound image U2 is superimposed on the still image of the ultrasound image U2 for display.

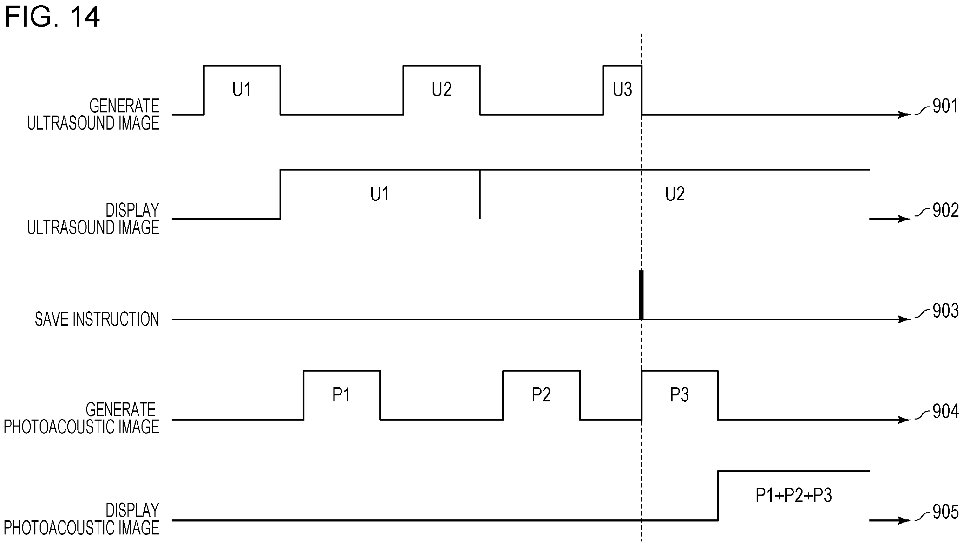

Fig. 14 is another timing chart in a case where a save instruction is received when the ultrasound image U2 is being displayed. In this case, the still image display of the ultrasound image U2 is continued when a save instruction is received. When a save instruction is received, the generation of the ultrasound image U3 is discontinued, and generation of the photoacoustic image P3 is started. When the generation of the photoacoustic image P3 completes, the ultrasound image U2 and a photoacoustic image P1+P2+P3 are saved in association. Then, the photoacoustic image P1+P2+P3 associated with the ultrasound image U2 is superimposed on the currently displayed ultrasound image U2 for display. Here, the photoacoustic image P1+P2+P3 is a composition image of the photoacoustic image P1, the photoacoustic image P2, and the photoacoustic image P3.

In the case illustrated in Fig. 14, the S/N ratio of a photoacoustic image can be improved more than the case in Fig. 13. Because a save instruction triggers to discontinue transmission and reception of ultrasonic waves for prioritizing reception of photoacoustic waves, the time interval from acquisition of the ultrasound image U2 to acquisition of the photoacoustic image P3 can be reduced.

### Third Embodiment

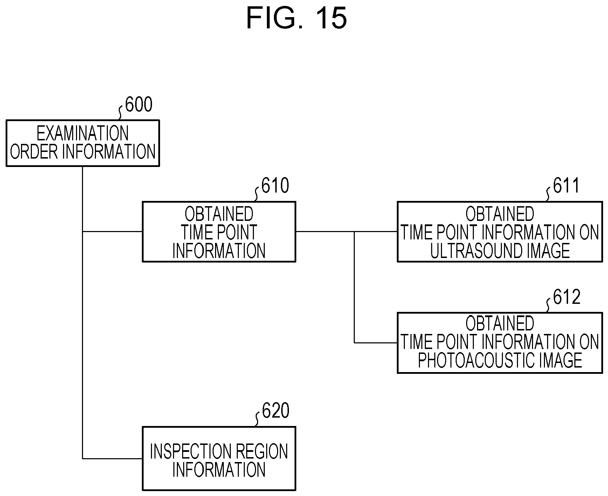

An inspection system according to a third embodiment determines images to be saved in association with each other based on examination order information transmitted from an external network such as an HIS or an RIS. Fig. 15 illustrates a data structure of examination order information 600 obtained by the inspection system according to this embodiment.

Information included in the examination order information 600 is directly input by a doctor, for example, by using an HIS or an RIS. Alternatively, an HIS or an RIS, for example, may generate information to be included in the examination order information 600 based on information input by a doctor, for example.

The examination order information 600 includes acquisition time point information 610. The acquisition time point information 610 is information representing at which time point an ultrasound image or a photoacoustic image is to be obtained with reference to the time point of a save instruction. The acquisition time point information 610 includes ultrasound image acquisition time point information 611 and photoacoustic image acquisition time point information 612. For example, the acquisition time point information 610 corresponds to information representing a relationship between a save instruction and an ultrasound image or a photoacoustic image to be saved as in the first or second embodiment.

The control unit 153 reads out the ultrasound image acquisition time point information 611 from the examination order information 600. The control unit 153 when receiving information representing a save instruction sets an ultrasound image acquisition time point corresponding to the time point of the save instruction based on the ultrasound image acquisition time point information 611. The control unit 153 determines an ultrasound image obtained at the set acquisition time point to be saved.

The control unit 153 reads out the photoacoustic image acquisition time point information 612 from the examination order information 600. The control unit 153 when receiving information representing a save instruction sets a photoacoustic image acquisition time point corresponding to the time point of the save instruction based on the photoacoustic image acquisition time point information 612. According to the first embodiment, the control unit 153 controls the probe 180 to irradiate light to the object 100 at the set acquisition time point. Then, the photoacoustic image obtained due to the light irradiation is determined to be saved. On the other hand, according to the second embodiment, the control unit 153 determines a photoacoustic image obtained at the set acquisition time point to be saved.

Thus, an ultrasound image and a photoacoustic image obtained based on the acquisition time point information 610 included in the examination order information 600 are stored in the storage unit 152. The acquisition time point information 610 read from the examination order information 600 is saved as the acquisition time point information 313 for the saved data 300.

The examination order information 600 may include inspection region information 620 which is information regarding a region to be inspected such as the head and the breast. The control unit 153 may read out the inspection region information 620 from the examination order information 600 and may set a predetermined ultrasound image or photoacoustic image acquisition time point for each inspection region based on the inspection region information 620. In this case, when the acquisition time point information 610 is not included in the examination order information 600, the control unit 153 can set an ultrasound image or photoacoustic image acquisition time point based on the examination order information 600. For example, the control unit 153 can read out an acquisition time point corresponding to an inspection region with reference to a relationship table describing correspondence between inspection region and acquisition time point, which is stored in the storage unit 152. The control unit 153 may obtain an acquisition time point based on any information included in examination order information, instead of the information regarding an inspection region, if the information is associated with an acquisition time point.

The control unit 153 settings a type of photoacoustic image to be generated based on the inspection region information 620, such as an oxygen saturation distribution set as the type of photoacoustic image to be generated, based on inspection region information 620 attached to the examination order information 600.

For example, the examination order information 600 may include information regarding the type of ultrasound image or photoacoustic image to be captured and the type of contrast agent to be used instead of the acquisition time point information 610. Additionally or alternatively, the examination order information 600 may include information regarding the type of probe for capturing an ultrasound image or a photoacoustic image, the position of the probe, an output to the probe such as voltage, and the sex, age, physical size, medical history, the number of weeks of pregnancy, and body temperature of an object.

The control unit 153 may compare saved data regarding a previously inspected object and the examination order information 600 and, for example, if the objects therein are matched, set an acquisition time point based on a previous inspection result.

In response to a save instruction given when a photoacoustic image is being displayed, an ultrasound image may be saved in addition to the photoacoustic image. The aforementioned first to third embodiments are based on a diagnosis with an ultrasound image and assume to provide information derived from a photoacoustic signal as additional information. On the other hand, this case is based on a diagnosis with a photoacoustic image and is assumed to use an ultrasound image as additional information. According to this case, a save instruction can be received when a photoacoustic image is being displayed, like a save instruction given when an ultrasound image is being displayed according to the first to third embodiments. The saving an ultrasound image and a photoacoustic image according to this case can be executed in the same manner as the saving based on information representing a save instruction according to the first to third embodiments. In other words, according to this case, an ultrasound image and a photoacoustic image (information derived from a photoacoustic signal) according to the first to third embodiments are interchanged. According to this case, when a user needs to save an ultrasound image when checking a photoacoustic image, both of an ultrasound image and a photoacoustic image can be saved, reducing the work of switching the display image to an ultrasound image. Thus, even after an inspection, the user can check a superimposition image of the photoacoustic image and the ultrasound image obtained with a small time difference therebetween.

### Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. An apparatus comprising:
first obtaining means (140, 180) configured to obtain an ultrasound image generated by transmitting and receiving ultrasonic waves to and from an object (100);
display control means (153) configured to control display means (160) to display the ultrasound image;
second obtaining means (140, 180) configured to obtain a photoacoustic signal generated by receiving photoacoustic waves generated from light irradiated to the object (100); and
saving control means (153) configured to obtain information representing a save instruction given when the ultrasound image is being displayed, and configured to save in storage means (152) the ultrasound image corresponding to a time point of the save instruction and information derived from the photoacoustic signal based on the information representing the save instruction.

2. The apparatus according to Claim 1, wherein the saving control means (153), based on the information representing the save instruction, is configured to save in the storage means (152) the ultrasound image corresponding to the time point of the save instruction and the information derived from the photoacoustic signal corresponding to the time point of the save instruction in association.

3. The apparatus according to Claim 1 or 2, wherein the saving control means (153), based on the information representing the save instruction, is configured to save in the storage means (152) the ultrasound image displayed when the save instruction is given and information derived from the photoacoustic signal obtained at a time point neighboring in time to the ultrasound image.

4. The apparatus according to Claim 1 or 2, wherein the saving control means (153), based on the information representing the save instruction, is configured to save in the storage means (152) the ultrasound image displayed when the save instruction is received and information derived from the photoacoustic signal obtained at a plurality of time points neighboring in time to the ultrasound image.

5. The apparatus according to Claim 1 or 2, wherein the saving control means (153), based on the information representing the save instruction, is configured to save in the storage means (152) the ultrasound image displayed when the save instruction is received and synthesized information of information pieces derived from the photoacoustic signals obtained at a plurality of time points neighboring in time to the ultrasound image.

6. The apparatus according to any one of Claims 1 to 5, wherein the second obtaining means (140, 180) generates a photoacoustic image as information derived from the photoacoustic signal by using the photoacoustic signal.

7. The apparatus according to Claim 6, wherein the saving control means (153) is configured to:
display a moving image of the ultrasound images on the display means (160); and
based on the information representing the save instruction, change the moving image to a still image of a superimposition image of the ultrasound image and the photoacoustic image corresponding to the time point of the save instruction for display on the display means (160).

8. The apparatus according to any one of Claims 1 to 7, wherein the saving control means (153) is configured to determine whether a region of interest is included in the ultrasound image by performing an image process, and, in a case where it is determined the region of interest is included in the ultrasound image, generate information representing the save instruction.

9. The apparatus according to any one of Claims 1 to 8, further comprising:
control means (150) configured to control light irradiating means (110) not to execute light irradiation to the object until information representing the save instruction is obtained,
wherein the control means (150) controls the light irradiating means (110) to execute light irradiation to the object after the save instruction based on the information representing the save instruction.

10. A method comprising:
obtaining an ultrasound image generated by transmitting and receiving ultrasonic waves to and from an object;
displaying the ultrasound image;
obtaining information representing a save instruction given when the ultrasound image is displayed;
obtaining a photoacoustic signal generated by receiving photoacoustic waves generated from light irradiated to the object; and
saving the ultrasound image corresponding to a time point of the save instruction and information derived from the photoacoustic signal corresponding to the time point of the save instruction based on the information representing the save instruction.

11. The method according to Claim 10, further comprising:
based on the information representing the save instruction, saving the ultrasound image corresponding to the time point of the save instruction and the information derived from the photoacoustic signal corresponding to the time point of the save instruction in association.

12. The method according to Claim 10 or 11, further comprising:
based on the information representing the save instruction, saving the ultrasound image displayed when the save instruction is given and information derived from the photoacoustic signal obtained at a time point neighboring in time to the ultrasound image.

13. The method according to Claim 10 or 11, further comprising:
based on the information representing the save instruction, saving the ultrasound image displayed when the save instruction is received and information derived from the photoacoustic signal obtained at a plurality of time points neighboring in time to the ultrasound image.

14. The method according to Claim 10 or 11, further comprising:
based on the information representing the save instruction, saving the ultrasound image displayed when the save instruction is received and synthesized information of information pieces derived from the photoacoustic signals obtained at a plurality of time points neighboring in time to the ultrasound image.

15. The method according to any one of Claims 10 to 14, further comprising:
generating a photoacoustic image as information derived from the photoacoustic signal by using the photoacoustic signal.

16. The method according to Claim 15, further comprising:
displaying a moving image of the ultrasound image; and
based on the information representing the save instruction, changing the moving image to a still image of a superimposition image of the ultrasound image and the photoacoustic image corresponding to the time point of the save instruction for display.

17. The method according to any one of Claims 10 to 16, further comprising:
determining whether a region of interest is included in the ultrasound image by performing an image process, and, in a case where it is determined the region of interest is included in the ultrasound image, generating information representing the save instruction.

18. The method according to any one of Claims 10 to 17, further comprising:
starting light irradiation to the object after the save instruction based on the information representing the save instruction.

19. A program that when runs on a computer causes the computer to execute the method according to any one of Claims 10 to 18.
